# EUROPEAN PATENT SPECIFICATION

(11) **EP 1 047 460 B1**
(45) Date of publication and mention of the grant of the patent: **19.10.2005**
(21) Application number: 98963305.2
(22) Date of filing: 23.12.1998
(51) Int. Cl.: A61L 27/00, A61L 27/06

(54) **POROUS NICKEL-TITANIUM ALLOY ARTICLE**
PORÖSER GEGENSTAND AUS EINER NICKEL-TITAN LEGIERUNG
ARTICLE POREUX EN ALLIAGE NICKEL-TITANE

(30) Priority: 31.12.1997 US 1496
(43) Date of publication of application: 02.11.2000
(73) Proprietor: Biorthex Inc., Montreal, Quebec H4N 3H5 (CA)
(72) Inventor: GJUNTER, Victor, Tomsk, 634050 (RU)
(74) Representative: Böhm, Brigitte
(86) International application number: PCT/CA1998/001202
(87) International publication number: WO 1999/034845

(56) References cited:
- WO-A-98/16267
- DE-A- 4 210 801
- US-A- 4 891 182
- SHABALOVSKAYA S A ET AL: "SURFACE SPECTROSCOPIC CHARACTERIZATION OF TINI NEARLY EQUIATOMIC SHAPE MEMORY ALLOYS FOR IMPLANTS" JOURNAL OF VACUUM SCIENCE AND TECHNOLOGY: PART A, vol. 13, no. 5, 1 September 1995, pages 2624-2632, XP000550454

## Description

### TECHNICAL FIELD

This invention relates to a porous article of a nickel-titanium alloy having medical and non-medical applications.

### BACKGROUND ART

Nickel-titanium alloys are known shape memory alloys which have been proposed for use in various environments including robotics and in memory devices of medical implants.

Soviet Union Patent Specification 1,381,764 dated 1982 proposes implants for facial reconstruction fabricated from a nickel-titanium alloy having a porosity of 8-60%, but the disclosure is very limited and there has been no commercial development of the material described.

U.S. Patents 4,2.06,516; 4,101,984; 4,017,911 and 3,855,638 all describe composite implants having a solid substrate with a thin porous surface coating. U.S. Patent 3,852,045 describes a bone implant element of porous structure in which the pores are developed by means of solid expendable void former elements which are arranged in a selected spatial pattern in a form cavity; metallic particles are packed about the void former elements, the mix is densified, the void former elements are removed, such as by vaporization and the metallic particles are sintered.

The search continues for materials suitable for fabricating medical implants, and for materials of improved physical characteristics.

### DISCLOSURE OF THE INVENTION

This invention seeks to provide a porous article of a nickel-titanium alloy, useful in biomedical and other applications.

In accordance with the invention there is provided a porous article based on a nickel-titanium alloy, the article having a porosity of at least 40% to 90% and said porosity being defined by a network of interconnected passageways extending throughout the article, the network exhibits a permeability for fluid material effective to permit complete migration of the fluid material throughout the network, and the article is elastically deformable.

### DESCRIPTION OF PREFERRED EMBODIMENTS

### i) Porous Alloy Article

The porous alloy article of the invention has a porosity of at least 40% to 90%, and more especially comprises a porous sintered powder body, in which the porosity extends throughout the body. In particular, the body may be formed with a controllable and variable porosity.

Preferably, the porosity is not more than 80%.

Preferably the permeability is derived from capillarity in the network ofpassageways which define the porosity.

This capillarity may be produced in the article by inclusion therein of a large number of pores of fine size which interconnect to produce capilliary passages.

Capillarity is advantageous in that it promotes migration of a desired fluid material into the network of passageways, and retention of the fluid material in the network, without the need to apply external hydraulic forces.

In general the network has a coefficient of permeability of 2 x 10⁻¹³ to 2 x 10⁻⁵, and the permeability is isotropic.

The capillarity and the isotropic character are, in particular, achieved when the network defining the porosity comprises pores of different pore size, the pore size distribution being as follows:

| Pore Size in Microns | Quantity |
|---|---|
| 10⁻² - 10⁻¹ | 1 - 5% |
| 10⁻¹ - 10 | 5 - 10% |
| 10 - 100 | 10 - 20% |
| 100 - 400 | 20 - 50% |
| 400 - 1000 | 10 - 50% |
| above 1000 | remainder to 100% |

In an especially preferred embodiment the pore size distribution is as follows:

| Pore Size in Microns | Quantity |
|---|---|
| 10⁻² - 10 | 5 - 15% |
| 10 - 400 | 15 - 70% |
| 400 - 1000 | 10 - 70% |
| above 1000 | remainder to 100% |

The porosity of a material affects its physio-mechanical qualities, for example, mechanical durability, corrosion resistance, superelasticity and deformational cyclo-resistivity.

The porous article of the present invention permits a wide field of application of the article without modifying the biomechanical and biochemical compatibility.

The size of the pores, the directional penetrability and the coefficient of wettability for biological fluids, as well as factors such as differential hydraulic pressure in the saturated and unsaturated porous article, determine the speed and adequacy of penetration of the biological fluid into the porous article.

It may be expected that an optimal pore size will provide permeability to the fluid and effective contact for bonding of components in the fluid with the interior pore surfaces of the article; the area of these surfaces depends on the pore sizes and the pore size distribution.

If the pore size is decreased the permeability changes unpredictably, since, on the one hand, the hydraulic resistance increases, while, on the other hand, the capillary effect appears at a certain low pore size, which capillary effect increases the permeability.

Pore size is also an important factor in tissue or biological aggregate growth. At least some of the pores need to be of a size to permit the development or growth of biological aggregates synthesized from the components of the fluid, for example, osteons, in the case of bone tissue.

Furthermore, if pore size is increased, the capillary effect decreases and the durability of the porous article also decreases. For each kind of live tissue there are optimum parameters of permeability, porosity and pore size distribution in the porous article for efficient operation of the porous article as an implant. The porous article of the invention functions well with a wide variety of live tissue and thus permits wide scope of use.

### ii) Nickel-Titanium Alloy

The porous nickel-titanium based alloy may suitably comprise 2 to 98%, by atomic weight, titanium and 2 to 98%, by atomic weight, nickel, to a total of 100%; preferably 40 to 60%, by atomic weight, titanium and 60 to 40%, by atomic weight, nickel to a total of 100%; and more preferably 48 to 52%, by atomic weight, titanium, 48 to 52%, by atomic weight, nickel, less than 2%, by atomic weight, molybdenum, less than 2%, by atomic weight, iron and minor or trace amounts of other elements, to a total of 100%. Desirably the alloy contains each of molybdenum and iron in an amount of more than 0%, by atomic weight and less than 2%, by atomic weight.

Nickel-titanium based alloys have significant advantages, as compared with other materials, in biomedical applications. In particular they display a high level of inertness or biocompatibility, and have high mechanical durability thus providing longevity when employed in the fabrication of implants.

Live tissue has an elasticity which renders it resilient to permanent deformity when subjected to stress and vibrations. If material employed in an implant which contacts such tissue has different characteristics from the tissue it will not meet the requirement for biomechanical compatibility in an implant and longevity will be short. The porous nickel-titanium alloy article of the invention is found to display mechanical behaviour very similar to that of live tissue, thus showing high biomechanical compatibility

### iii) Article

Various porous articles may be fabricated in accordance with the invention.

One especially preferred class of articles of the invention is biomedical implants.

The porous alloy article may be fabricated as an implant or endoprosthesis for local or total replacement of a body part, for example, to correct birth defects or defects resulting from injury or disease.

Thus the porous alloy article may be fabricated as a spacer to replace a portion of shattered human bone and provide a bridge for connection of bone parts separated as a result of the shattering of the original bone.

Nickel-titanium based alloys have a high level of biocompatibility with human tissue and the capilliarity of the porous alloy article of the invention facilitates penetration of the article by human biological fluids under the force of capillary action. Thus in the case of a porous spacer fabricated from the porous article of the invention, biological fluid from the bone is drawn into the network of passageways of the contacting porous spacer, and the fluid migrates, under capillary action, throughout the network. Live tissue in the fluid grows within the pores of the network and adheres to the pore surfaces providing a chemical bonding or unification with the titanium-nickel based alloy.

As the growth of tissue, for example, bone, is completed there is provided both a chemical bonding between the newly grown bone and the titanium nickel spacer, and a mechanical connection by the newly grown bone, to the separated bone parts which the spacer bridges.

The porous article may also be employed in other applications, for example, in a flame torch, where the porous article is fabricated as the torch head to provide a desired flame formation. A further application is as the working element of a surgical tool where the porous structure may act as a reservoir for a cooling liquid where the tool is employed in cryosurgery. In the case where the working element is a cutting edge or tip, the cooling liquid in the porous structure may provide local freezing at a site where a cut or incision is being made, such as in wart removal.

Other applications of the porous article include a deformable filtering element for liquid and gas environments.

### iv) Process

The porous article is produced with a controlled and variable pore size distribution, as indicated above. In particular the porous article may be produced in accordance with the procedures described in the Russian publication "Medical Materials and Implants with Shape Memory Effect", 1998, Tomsk University, p-460 to 463, Gunther V. et al. In summary, this alloy can be produced by powder metallurgy method by means of the so-called SHS (Self-Propagating High-Temperature Synthesis) method using two different processes:
- Layerwise combustion SHS: Heat is generated by initial ignition. Then thermal conductivity raises the temperature of the neighbouring layers of the substance, thus causing reaction within, and thus resulting in spatial displacement of the reaction zone in the volume. The reaction takes place only in a thin layer called the combustion front.
- Thermal shock SHS: In this process, the thermal shock is performed by heating a mixture of various powders up to the temperature at which the self supported chemical reaction and heat release effect takes place. Due to the self-heating process the mixture is heated up to higher temperatures, thus the mixture of the powders is converted into alloy.

The two processes are explained more fully hereinafter:

Self-propagating High Temperature Synthesis (SHS) is based on the use of the heat which is emitted under interaction (exothermal reaction) of the various elements, in particular different metals. There are two distinguished processes: SHS in condition of layerwise combustion and SHS in the condition of thermal shock.

In accordance with the layerwise combustion, the heat is emitted in a certain local volume of the substance due to excitation of the exothermal reaction. This heat, by mean of thermal conductivity, raises the temperature of the neighbouring layers of the substance thus causing reaction within and thus resulting in spatial displacement of the reaction zone in the volume. Under layerwise combustion the chemical reaction does not take place in the whole substance volume simultaneously. It takes place only in the thin layer called the combustion front.

In the SHS, the thermal shock process, the rise of the temperature in the whole volume of the reacting system results in a time extended self-heating procedure which is similar to thermal explosion.

The SHS in layerwise combustion is performed as follows. A blank of predetermined shape, e.g., cylindrical, is pressed from a mixture of the powders of the various metals (alloys). A thermal impulse is supplied towards a top or bottom section thus igniting the cylinder. The chemical reaction is initiated in a surface layer and spontaneously propagates in the form of the combustion front which travels along the blank axis. This combustion front leaves behind a solidified cold product (alloy).

SHS in the condition of thermal shock is performed by heating a mixture of various powders up to the temperature at which the self-supported chemical reaction and heat release effect takes place. Due to the self-heating process the mixture is heated up to a high temperature and the mixture of powders is converted into alloy.

During titanium nickel synthesis in conditions of the thermal shock and layerwise combustion the maximum temperatures of the reaction zone are similar and they are determined with energetic capabilities of the system. These temperatures can be lower, equal or somehow higher than the smelting temperature of the titanium-nickel alloy.

In layerwise and thermal shock conditions the main SHS parameters of titanium-nickel production are determined with thermodynamic analysis, employing the laws and formation mechanisms of titanium-nickel in nonexothermal conditions. The most suitable parameters to control the synthesis are initial temperature of the process, the holding time of the initial composition under given temperature, the degree of mixing of the composition for a final product and type of the initial powders. These parameters control the pores size distribution in the porous material. Two types of product, the titanium-nickel ingot and sintered porous titanium nickel, can be obtained with varying of the referred parameters. It has to be noted the reaction execution in the case of ingot is higher.

The optimal conditions to obtain nickel-titanium alloys by the SHS process in regimes of layerwise combustion and thermal shock are as follows:
i) the initial synthesis temperature in layerwise combustion conditions to produce the porous briquette is suitably 500 to 900°K;
ii) the initial synthesis temperature in thermal shock conditions is close to the melting point of low-melting eutectic (1200K);
iii) the inert gas pressure in a closed reactor is 1 to 2 x 10⁵ Pa;
iv) the inert atinosphere with no pressure is supported in the reactor of the open type;
v) the gas feed rate is about 0.2-0.4 atm/min.;
vi) the holding time of the initial mixtures under the synthesis temperature or close to it depends on the type of the used powders and on blank (or sintered bar) diameter and usually it is equal 0.5-2 hr.;
vii) when the blank diameter is increased, the other parameters are constant, the synthesis temperature should be lowered;
viii) the increase of the density of the initial mixture requires the reduction of the initial temperature of the synthesis;
ix) the minimum blank diameter is 10 mm (in accordance with the purpose of reducing of the heat losses and approaching the adiabatic combustion conditions);
x) initial blank porosity is about 30-60% (here the blank is easily ignited and combustion process is going in a stable manner);
xi) the lower level of porosity may result in interruption of the combustion process due to substantial heal release out of the synthesis zone, the higher level of porosity results in lower mechanical strength of the blank;
xii) the remaining parameters such as: particle sizes and type of the initial powders, concentration of the main and alloying elements are varied with the object of production of material of predetermined composition and properties. Suiteably the particle size of the powders is 5 to 100 µ

The technological production of nickel-titanium alloys suitably employs three stages.
Stage 1. Manufacturing of the blank to be used for SHS process.
   a) **Drying of the powders.** Usually the powdered components are dried in a vacuum chamber under a temperature of 350-360°K. The holding of the powders at this temperature for about 7 hours is sufficient for removal of the moisture and for reliable ignition and further combustion of the mixture.
   b) **The metering of the components**. The main components are metered with an accuracy of ± 20 mg, for alloying components this value is suitably within the accuracy of ± 0.1 mg. The standard balance devices may be used when a relatively large number of mixtures is produced.
   c) **Mixing.** The powders are mixed in accordance with usual procedures inside a standard mixer during 6-8 hours. In high energy mixers (attritors) the time of mixing is reduced substantially.
   d) **Briquettes manufacturing.** The cylindrical briquettes are pressed in the press moulds with a hydraulic press machine. Also there can be used the packing of forms that are filled with the powder mixture.
Stage 2. Self Propagating High Temperature Synthesis.
   a) **Loading and preparation of the reactor for performing the SHS** Process. In a simple embodiment a reactor is employed comprising a tube made of stainless steel with couplings for inert gas and thermocouples. A side opening of this tube is mounted with electric coil to ignite the mixture. In order to produce sized briquettes or consumable electrodes, the reactor is filled with a mixture of the metal powders, which is compacted while loading. The pressed briquettes are placed inside the reactor. The reactor is ventilated with inert gas.
   b) **Preheating and Ignition.** If SHS process is not performed at room temperature, the initial temperature is raised. For example, a preheating of the briquettes is applied. The initial synthesis temperature is monitored with thermocouples. In order to obtain porous sintered products, the initial process temperature in condition of the layerwise combustion is suitably 500-900°K. In case of constant initial temperature of the synthesis, synthesis time and mixture composition, there is a possibility to control the pores distribution with dimensions inside of the final product due to temperature gradient that takes place across the diameter of the sintered bar.
   c) **Cooling and Unloading of the SHS Products**. When the combustion front has passed, the reactor is evacuated from the furnace and it is cooled with water down to a temperature of 340-360°K without stopping the supply of inert gas. Subsequently the reactor is unloaded with synthesised product - porous briquette or bar, depending on synthesis conditions. The resulting article may be used as is or subjected to further processing.
Stage 3. The production of the semifinished products, for example, ingots, rods, wire or rolled stock is performed with known methods.

Following the above procedure the titanium-nickel alloy article is porous. During the synthesis process the total porosity has a tendency to be constant, but there takes place a loosening of material. Redistribution of porosity is caused by formation and leaking of the liquid phase, and also by reaction of adsorbed gases that are filtered through the reacted part of the sample.

Under all initial temperatures of the synthesis the porous material contains the phases of TiNi and Ti₂Ni. The last one in form of segregation is presented in small amounts at the grain boundaries. With increase of the initial synthesis temperature, the content of the phase Ti₂Ni is decreased and correspondingly the strength of material is higher. The porous titanium nickel has a controllable porosity 8-90% and a pore size distribution as described above. In special circumstances the porous structure of titanium nickel is processed with acids in order to increase the porosity and penetration factor. The nickel-titanium article has superior elastic properties and thermo-mechanical memory.

### BRIEF DESCRIPTION OF DRAWINGS

Fig. 1 shows a porous article of the invention as an implant;
Fig. 2 shows the microstructure of an implant surface with in-grown tissue, 9 months after surgery; and
Fig. 3 shows an implant for facial skull.

### DESCRIPTION WITH REFERENCE TO DRAWINGS

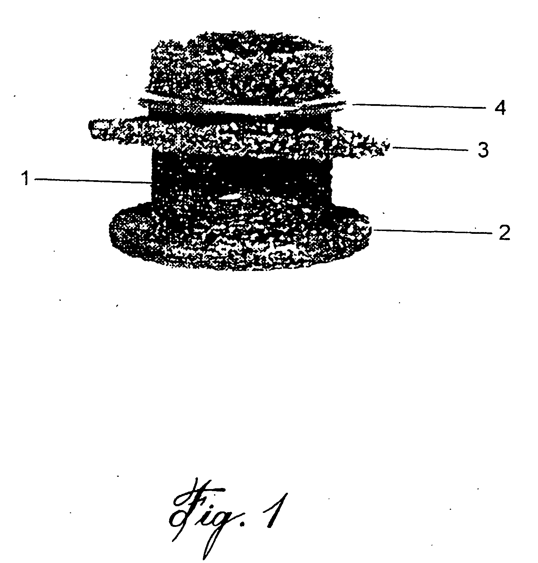

With further reference to Fig. 1 a porous titanium-nickel alloy implant for through wounds of the liver comprises a sleeve 1, immobile limiting rib 2, removable limiting rib 3 and a retaining ring 4.

### EXAMPLE 1

### Implant operation of a through wound of the liver.

Nickel-titanium alloy having equal amounts of nickel and titanium, by atomic weight percent and a porosity of 65% was used, the alloy had an isotropic permeability of 2 x 10⁻⁵ m² and a distribution of porosity according to the following pore sizes:

| | |
|---|---|
| 10⁻²-10⁻¹ microns | 5% |
| 10⁻¹ -10 | 10% |
| 10 - 100 | 20% |
| 100 - 400 | 50% |
| 400 - 1000 | 10% |
| over 1000 | remainder to 100% |

The composition was obtained by powder metallurgy method by means of a self-propagating high temperature synthesis regime of combustion with etching or pickling afterwards. The initial process conditions were: temperature = 550K, powder porosity = 59%. The porous material for the implant was made in a configuration of sleeve 1 (Fig. 1) having spaced apart limiting ribs 2, 3, one rib 2 being stationary and the other 3 being removable. The diameter of the sleeve 1 was 20 mm, length 30 mm, outside diameter of ribs 2, 3 was 25 mm. The removable rib 3 was mounted by means of a retaining ring 4.

The implant was utilised as follows:

After sterilisation treatment, the animal (a dog) was anaesthetised to allow access to the left lateral hemisphere of the liver by means of intermediate "lipothymy" using a sword-like appendix 15 cm long. Then, by using a cylindrical resector with an outside diameter of 22 mm, a perforation imitating a wounding channel was effected in the liver. By means of a sleeve-conductor, sleeve 1 was inserted into the wounding channel until the limiting stationary rib 2 abutted the liver, whereas the second end of the sleeve 1 remained outside the liver. Then, the removable rib 3 was mounted on this second end of the sleeve 1 to the limit where the liver became slightly depressed in such position. Then, the retaining ring 4 was affixed on the sleeve 1. During the operation, the length of resection lasted 5 seconds, instalment and affixing of the implant lasted around 20 seconds; in this case, the bleeding of the liver happened only during resection and installation of implant. Blood loss was not more than 30 ml.

After installation of the implant, porous material was saturated with body liquid during the next 30-40 seconds, including the penetration of blood outside before it clotted inside the matrix of the pores. Blood loss during this operation was around 10-15 ml.

In the post-operation stage, during 12 months, the following occurrences were noticed: the dynamic of development and transformation of tissue on the border of the "parenchyma implant" including the development of a layer of fibrin (24 hours), its granulation (5-7 days), development and densening of collagen tissue in which vessels and liver lattice (6-12 months) had been developed. Thirty animals (dogs) were subjected to the described operation. Mortality rate in this group was 10% and was not implant-related, which is testimony of the high efficiency of the porous alloy material, which may be used for implantation of parachimose defects in human body organs.

### EXAMPLE 2

### Forming of Stump of the Eyeball.

For this operation, porous titanium-nickel alloy having equal amounts of nickel and titanium, by atomic weight percent and with a degree of porosity of 90%, isotropic permeability of 2 x 10⁻⁵ m², and the following distribution of pores according to sizes was used:

| | |
|---|---|
| 10⁻² -10⁻¹ microns | 1% |
| 10⁻¹ -10 | 5% |
| 10 - 100 | 10% |
| 100 - 400 | 20% |
| 400 - 1000 | 50% |
| over 1000 | remainder to 100% |

The process was the same as in example 1 but the initial condition were: temperature = 650K, powder porosity = 62%, the resulting alloy had an 80% porosity and further etching process provided the final 90%. The material was used with a cosmetic purpose and its effectiveness was determined according to the strength of bonding between the implant and the eyeball tissue, ability to resist resolving, and to maintain a predetermined volume. The following procedure was used: first, the lab animal (dog) was subjected to a visceroeniculation and an implant was placed in the scleral glass/goblet after all vessel tissue had been removed; then, the scleras between the eye movement muscles were severed and used as patches to affix the implant with paired stitches.

Twenty animals were subjected to this operation; throughout the procedure, the animals did not have any complications in the implant-affected areas or elsewhere.

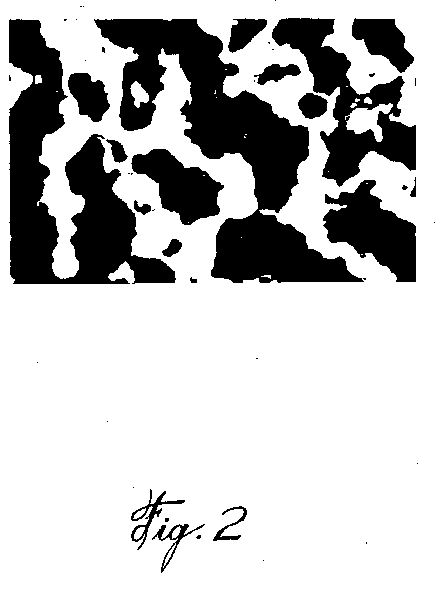

Bonding of tissue in the implant pores and its transformation was studied by means of a micro-section after 7 days, and 3, 6, 9 and 12 months after the operation. Fig. 2 shows microstructure of the microsectional surface of implant showing the tissue filling 9 months after the operation. The results of the study show that a dense fibrose tissue is created inside the pores which remains stable thus creating a secure fixation and functionality of the implant.

### EXAMPLE 3

### Implant of Facial Skull Structure.

During implantation of bone defects caused by osteoporosis (pathological disintegration of bone tissue), the most efficient titanium nickel alloy used was the one having equal amounts of nickel and titanium, by atomic weight percent and with a degree of porosity of 70-80%, isotropic permeability of 1.5 x 10⁻⁵ m² and the following distribution of pore sizes:

| | |
|---|---|
| 10⁻² - 10⁻¹ microns | 1% |
| 10⁻¹ -10 | 5% |
| 10-100 | 15% |
| 100 - 400 | 45% |
| 400 - 1000 | 30% |
| over 1000 | remainder to 100% |

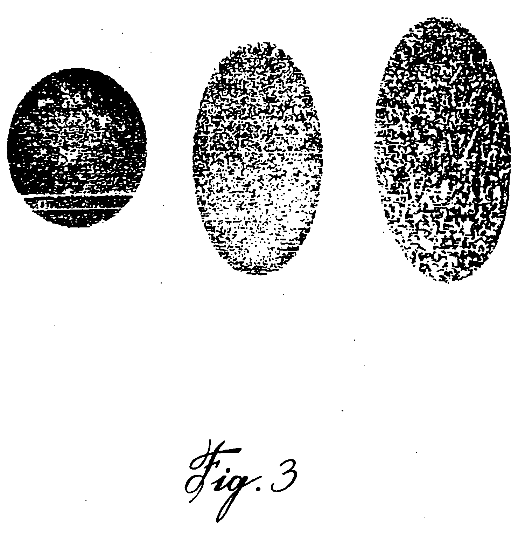

The material was obtained according to powder metallurgy method by means of self-propagating high temperature synthesis in a combustion regime. The initial process conditions were: temperature = 650K, powder porosity 62%. The material was tailored as a plate-like endoprothesis (Fig. 3) having a thickness from 0.5-1.0 mm obtained by the method of electro-erosion cutting of nuggets of porous titanium nickel alloy. Preparation of the endoprothesis for implantation consisted of sterilization by known standard procedures including dry-heat treatment and preservation in 96% alcohol.

Similar procedures for use of the porous material were used for replacement or implantation of other nose defects, frontal sinuses bone tissue and upper jaw tissue and lower wall of the eyeball cavity.

Results of the implants using porous titanium nickel alloy show significant advantages of the present material over known prior materials.

Material with a degree of porosity over 90% is today impractical in view of its low mechanical durability.

## Claims

1. A porous article based on a nickel-titanium alloy, said article having a controllable and variable porosity of at least 40% to 90% and said porosity being defined by a network of interconnected passageways extending throughout said article, said network exhibiting a permeability for fluid material effective to permit complete migration of the fluid material throughout said network, said article being elastically deformable.

2. A porous article according to claim 1, wherein said alloy is a biocompatible alloy and has biomechanical compatibility with human tissues.

3. A porous article according to claim 1 or 2, wherein said permeability arises from capilliarity effect in said network.

4. A porous article according to claim 1, 2 or 3, wherein said network is permeable to biological tissue in a biological fluid, and said network comprises pores of a size to permit growth of biological tissue therein.

5. A porous article according to claim 1, 2, 3, or 4, having a porosity of not more than 80%.

6. A porous article according to claim 1, 2, 3, 4, or 5, having a coefficient of permeability of 2 x 10⁻¹³ to 2 x 10⁻⁵.

7. A porous article according to claim 1, 2, 3, 4, 5, o r 6, comprising a porous sintered powder body.

8. A porous article according to any one of claims 1 to 7, wherein said permeability is isotropic.

9. A porous article according to any one of claims 1 to 8, wherein said network comprises a distribution of pore size as follows:
| Pore Size in Microns | Quantity |
|---|---|
| 10⁻² - 10⁻¹ | 1-5% |
| 10⁻¹ - 10 | 5-10% |
| 10 - 100 | 10-20% |
| 100 - 400 | 20 - 50% |
| 400 - 1000 | 10 - 50% |
| above 1000 | remainder to 100%. |

10. A porous article according to any one of claims 1 to 8, wherein said network comprises a distribution of pore size as follows:
| Pore Size in Microns | Quantity |
|---|---|
| 10⁻² - 10 | 5 - 15 % |
| 10 - 400 | 15 - 70% |
| 400 - 1000 | 10 - 70% |
| above 1000 | remainder to 100%. |

11. A porous article according to any one of claims 1 to 10, wherein said alloy comprises 2 to 98%, by atomic weight, titanium and 2 to 98%, by atomic weight, nickel, to a total of 100%.

12. A porous article according to any one of claims 1 to 10, wherein said alloy comprises 40 to 60%, by atomic weight, titanium and 60 to 40%, by atomic weight, nickel to a total of 100%.

13. A porous article according to any one of claims 1 to 10, wherein said alloy comprises 48 to 52%, by atomic weight, titanium, 48 to 52%, by atomic weight, nickel, less than 2%, by atomic weight, molybdenum, less than 2%, by atomic weight, iron and minor amounts of other elements, to a total of 100%.

14. A porous article according to any one of claims 1 to 13, in the form of a medical product application.

15. A porous article according to any one of claims 1 to 13, in the form of a technical product application.

## Patentansprüche

1. Poröser Artikel auf Basis einer Nickel-Titan-Legierung, wobei der Artikel eine kontrollierbare und variable Porosität von wenigstens 40% bis 90% aufweist und die Porosität durch ein Netzwerk von miteinander verbundenen Durchgängen die sich durch den Artikel erstrecken definiert ist, wobei das Netzwerk eine Durchlässigkeit für fluides Material aufweist, um eine vollständige Wanderung des fluiden Materials durch das Netzwerk zu erlauben, wobei der Artikel elastisch deformierbar ist.

2. Poröser Artikel nach Anspruch 1, wobei die Legierung eine biokompatible Legierung ist und biomechanische Kompatibilität mit menschlichen Geweben aufweist.

3. Poröser Artikel nach Anspruch 1 oder 2, wobei die Permeabilität auf einen Kapillareffekt in dem Netzwerk zurückzuführen ist.

4. Poröser Artikel nach Anspruch 1, 2 oder 3, wobei das Netzwerk für biologisches Gewebe in einem biologischen Fluid durchlässig ist, und wobei das Netzwerk Poren mit einer solchen Größe umfasst, um das Wachstum von biologischem Gewebe darin zu erlauben.

5. Poröser Artikel nach Anspruch 1, 2, 3 oder 4, der eine Porosität von nicht mehr als 80% aufweist.

6. Poröser Artikel nach Anspruch 1, 2, 3, 4 oder 5, der einen Durchlässigkeitskoeffizienten von 2 x 10⁻¹³ bis 2 x 10⁻⁵ aufweist.

7. Poröser Artikel nach Anspruch 1, 2, 3, 4, 5 oder 6, der einen porösen, gesinterten Pulver-Körper umfasst.

8. Poröser Artikel nach einem der Ansprüche 1 bis 7, wobei die Durchlässigkeit isotrop ist.

9. Poröser Artikel nach einem der Ansprüche 1 bis 8, wobei das Netzwerk eine Porengrößenverteilung wie folgt umfasst:
| Porengröße in µm | Menge |
|---|---|
| 10⁻² - 10⁻¹ | 1-5 % |
| 10⁻¹ - 10 | 5-10 % |
| 10 - 100 | 10-20 % |
| 100 - 400 | 20-50 % |
| 400 -1000 | 10-50 % |
| über 1000 | Rest bis 100 % |

10. Poröser Artikel nach einem der Ansprüche 1 bis 8, wobei das Netzwerk eine Porengrößenverteilung wie folgt umfasst:
| Porengröße in µm | Menge |
|---|---|
| 10⁻² - 10 | 5-15 % |
| 10 - 400 | 15-70 % |
| 400 - 1000 | 10-70 % |
| über 1000 | Rest bis 100 % |

11. Poröser Artikel nach einem der Ansprüche 1 bis 10, wobei die Legierung 2 bis 98 % Titan pro Atomgewicht und 2 bis 98 % Nickel pro Atomgewicht bis insgesamt 100 % aufweist.

12. Poröser Artikel nach einem der Ansprüche 1 bis 10, wobei die Legierung 40 bis 60 % Titan pro Atomgewicht und 60 bis 40 % Nickel pro Atomgewicht bis insgesamt 100 % aufweist.

13. Poröser Artikel nach einem der Ansprüche 1 bis 10, wobei die Legierung 48 bis 52 % Titan pro Atomgewicht, 48 bis 52 % Nickel pro Atomgewicht, weniger als 2 % Molybdän pro Atomgewicht, weniger als 2 % Eisen pro Atomgewicht und kleinere Mengen an anderen Elementen bis insgesamt 100 % aufweist.

14. Poröser Artikel nach einem der Ansprüche 1 bis 13, in Form einer medizinischen Produktanwendung.

15. Poröser Artikel nach einem der Ansprüche 1 bis 13, in Form einer technischen Produktanwendung.

## Revendications

1. Un article poreux à base d'alliage de nickel et de titane, ledit article possédant une porosité contrôlable et variable d'au moins 40% à 90%, ladite porosité étant définie par un réseau de passages interconnectés s'étendant à travers ledit article, ledit réseau démontrant une perméabilité envers un matériel fluide pouvant permettre une migration complète du matériel fluide à travers ledit réseau, ledit article étant déformable d'une manière élastique.

2. Un article poreux selon la revendication 1, dans lequel ledit alliage est un alliage biocompatible et possède une compatibilité biomécanique avec les tissus humains.

3. Un article poreux selon la revendication 1 ou 2, dans lequel ladite perméabilité provient d'un effet de capillarité dans ledit réseau.

4. Un article poreux selon la revendication 1, 2 ou 3, dans lequel ledit réseau est perméable au tissus biologique dans un fluide biologique, et ledit réseau comprend des pores de dimension telle qu'ils permettent d'y faire croître un tissus biologique.

5. Un article poreux selon la revendication 1, 2, 3 ou 4, possédant une porosité qui ne dépasse pas 80%.

6. Un article poreux selon la revendication 1, 2, 3, 4, ou 5, possédant un coefficient de perméabilité de 2 x 10⁻¹³ à 2 x 10⁻¹⁵.

7. Un article poreux selon la revendication 1, 2, 3, 4, 5, ou 6, comprenant une masse poreuse de poudre frittée.

8. Un article poreux selon l'une quelconque des revendications 1 à 7, dans lequel ladite perméabilité est isotrope.

9. Un article poreux selon l'une quelconque des revendications 1 à 8, dans lequel ledit réseau comprend une distribution de dimensions de pores comme suit :
| Dimension de pores en microns | Quantité |
|---|---|
| 10⁻² - 10⁻¹ | 1 - 5% |
| 10⁻¹ - 10 | 5 - 10% |
| 10 - 100 | 10 - 20% |
| 100 - 400 | 20 - 50% |
| 400 - 1000 | 10 - 50% |
| au delà de 1000 | ce qui reste jusqu'à 100%. |

10. Un article poreux selon l'une quelconque des revendications 1 à 8, dans lequel ledit réseau comprend une distribution de dimensions de pores comme suit :
| Dimension de pores en microns | Quantité |
|---|---|
| 10⁻² - 10 | 5 - 15% |
| 10 - 400 | 15 - 70% |
| 400 -1000 | 10 - 70% |
| au delà de 1000 | ce qui reste jusqu'à 100%. |

11. Un article poreux selon l'une quelconque des revendications 1 à 10, dans lequel ledit alliage comprend 2 à 98%, en poids atomique, de titane et 2 à 98%, en poids atomique, de nickel, pour un total de 100%.

12. Un article poreux selon l'une quelconque des revendications 1 à 10, dans lequel ledit alliage comprend 40 à 60%, en poids atomique, de titane et 60 à 40%, en poids atomique, de nickel, pour un total de 100%.

13. Un article poreux selon l'une quelconque des revendications 1 à 10, dans lequel ledit alliage comprend 48 à 52%, en poids atomique, de titane, 48 à 52%, en poids atomique, de nickel, moins de 2%, en poids atomique, de molybdène, moins de 2%, en poids atomique, de fer et des quantités mineures d'autres éléments, pour un total de 100%.

14. Un article poreux selon l'une quelconque des revendications 1 à 13, sous forme de produit d'application médicale.

15. Un article poreux selon l'une quelconque des revendications 1 à 13, sous forme d'application technique.
